# FASCICULE DE BREVET EUROPEEN

(11) **EP 1 292 572 B1**
(45) Date de publication et mention de la délivrance du brevet: **18.02.2004**
(21) Numéro de dépôt: 01940617.2
(22) Date de dépôt: 29.05.2001
(51) Int. Cl.: C07C 311/08, C07D 295/18, A61K 31/216, A61K 31/4965, A61P 13/00, A61P 1/00

(54) **PROPANOLAMINOTETRALINES, LEUR PREPARATION ET COMPOSITIONS PHARMACEUTIQUES EN CONTENANT**
PROPANOLAMINOTETRALINE, VERFAHREN ZU IHRER HERSTELLUNG SOWIE DIESE VERBINDUNGEN ENTHALTENDE PHARMAZEUTISCHE ZUSAMMENSETZUNGEN
PROPANOLAMINOTETRALINES, PREPARATION THEREOF AND COMPOSITIONS CONTAINING SAME

(30) Priorité: 06.06.2000 FR 0007190
(43) Date de publication de la demande: 19.03.2003
(73) Titulaire: SANOFI-SYNTHELABO, 75013 Paris (FR)
(72) Inventeur: BARZAGHI, Laura, I-20052 Monza (IT); BOVY, Philippe, R., F-78750 Mareil Marly (FR); CECCHI, Roberto, I-26900 Lodi (IT); CROCI, Tiziano, I-20156 Milano (IT); ROMAGNANO, Stefano, I-20090 Buccinasco (IT)
(74) Mandataire: Varady, Peter
(86) Numéro de dépôt international: PCT/FR2001/001652
(87) Numéro de publication internationale: WO 2001/094307

(56) Documents cités:
- EP-A- 0 714 883
- EP-A- 0 882 704
- EP-A- 0 976 720

## Description

La présente invention concerne de nouvelles phénoxypropanolamines, les compositions pharmaceutiques les contenant, un procédé pour leur préparation et des intermédiaires dans ce procédé.

WO99/51564 décrit des dérivés de la propanolamine de formula générale 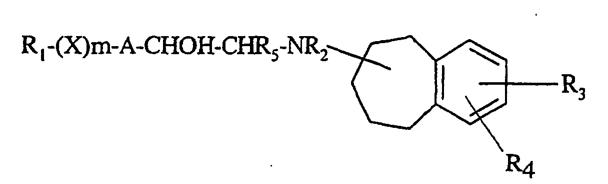 dans laquelle R₁ est un aryle, un hétérocycle ou un cycloalkyle, ces composés ayant une activité agoniste vis-à-vis des récepteurs bêta-3 adrénergiques et étant susceptibles d'être utilisés dans le traitement de nombreuses maladies telles que l'ulcère, la pancréatite, l'obésité, l'incontinence urinaire et la pollakiurie.

EP 0 375 560 décrit des aryloxypropanolaminotétralines ayant également une activité vis-à-vis des des récepteurs bêta-3 adrénergiques mais exerçant un effet antagoniste sur ces récepteurs. Certaines phénoxypropanolamines ayant une activité agoniste sur les récepteurs bêta 3 adrénergiques ont été décrites dans EP-A-0 714 883 et EP-A-0 976 720.

Il a été maintenant trouvé que certaines phénoxypropanolaminotétralines ayant une structure proche des composés décrits dans EP 0 375 560 possèdent une activité agoniste vis-à-vis des récepteurs bêta-3 adrénergiques.
Ainsi, la présente invention concerne, selon un de ses aspects, des phénoxypropanolamines de formule (I) 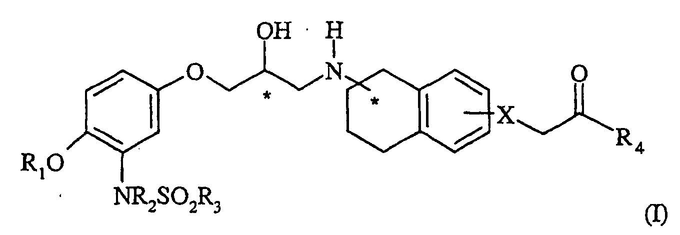 dans laquelle
- R₁ et R₂: représentent indépendamment un atome d'hydrogène, un groupe benzyle, un groupe benzoyle, un groupe -CO(C₁-C₄)Alk, un groupe -CH₂OCH₃, un groupe -COO(C₁-C₄)Alk ou un groupe benzyloxycarbonyle; ou bien
- R₁ et R₂: forment ensemble un groupe carbonyle, méthylène ou di(C₁-C₄)Alkméthylène pour constituer une structure hétérocyclique avec les atomes d'oxygène et d'azote qui les portent ;
- R₃: représente un atome d'hydrogène ou un groupe (C₁-C₄)Alk ;
- R₄: représente un groupe hydroxy, un groupe (C₁-C₄)alcoxy, ou un groupe NR₅R₆ ;
- R₅ et R₆: représentent indépendamment un atome d'hydrogène; un groupe (C₁-C₄)Alk; un groupe aryle ou hétéroaryle éventuellement substitué par un groupe R₇ ; un groupe aralkyle ou hétéroaralkyle éventuellement substitué par un groupe R₇ ; ou bien R₅ et R₆, avec l'atome d'azote qui les porte, forment un cycle de 5 à 7 atomes, saturé ou insaturé ;
- R₇: représente un atome d'hydrogène ou d'halogène, un groupe hydroxy, un groupe (C₁-C₄)Alk , un groupe (C₁-C₄)alcoxy, -COOH, -COO(C₁-C₄)Alk, - CN, -NH(C₁-C₄)Alk ou -N(C₁-C₄)Alk₂ ;
- X: représente O ou CH₂ ;
et leurs sels ou solvates.

Dans la présente description le terme (C₁-C₄)Alk désigne un radical monovalent d'un hydrocarbure en C₁-C₄ saturé à chaîne droite ou ramifiée.

Dans la présente description le terme halogène désigne un atome choisi parmi le chlore, le brome le iode et le fluor.

Les groupes aryles ou hétéroaryles comprennent notamment le phényle, le naphtyle et le pyridyle.

Les groupes aralkyles ou hétéroaralkyles comprennent notamment le benzyle, le naphtylméthyle et le pyridylméthyle.

Les cycles de 5 à 7 atomes, saturés ou insaturés, comprennent notamment la pyrrolidine, la pipéridine, la morpholine et la thiomorpholine.

Un groupe -COO(C₁-C₄)Alk préféré est le *tert*-butoxycarbonyle (Boc).

Des composés préférés comprennent les composés où le substituant -X-CH₂-CO-R₄ est rattaché à la position 7 de la tétraline.

Des composés préférés comprennent les composés où R₃ représente groupe (C₁-C₄)Alk.

Les sels des composés de formule (I) selon la présente invention comprennent aussi bien les sels d'addition avec des acides minéraux ou organiques pharmaceutiquement acceptables tels que le chlorhydrate, le bromhydrate, le sulfate, l'hydrogénosulfate, le dihydrogénophosphate, le citrate, le maléate, le tartrate, le fumarate, le gluconate, le méthanesulfonate, le 2-naphtalènesulfonate, etc., que les sels d'addition qui permettent une séparation ou une cristallisation convenable des composés de formule (I) tels que le picrate, l'oxalate ou les sels d'addition avec des acides optiquement actifs, par exemple les acides camphosulfoniques et les acides mandéliques ou mandéliques substitués.

De plus, lorsque les composés de formule (I) possèdent un groupe carboxy libre les sels comprennent aussi les sels avec des bases minérales, de préférence celles avec des métaux alcalins tels que le sodium ou le potassium, ou avec des bases organiques.

Les stéréoisomères optiquement purs, ainsi que les mélanges d'isomères des composés de formule (I), dus aux carbones asymétriques, dans une proportion quelconque, sont partie de la présente invention.

Des composés de formule (I) préférés sont les composés où la configuration du carbone de la propanolamine portant le groupe OH est (S).

D'autres composés de formule (I) préférés sont les composés où la configuration du carbone de la tétraline lié au groupe amino est (S).

Des composés de formule (I) particulièrement préférés sont les composés où la configuration des carbones marqués avec " * " est (S).

D'autres composés préférés sont les composés où la tétraline est rattachée au groupe amino en position bêta.

Les composés de formule (I) peuvent être préparés en traitant un composé de formule (II): 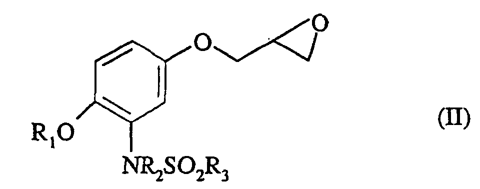 dans laquelle R₁, R₂ et R₃ sont tels qu'indiqués ci-dessus, avec une amine de formule (III) 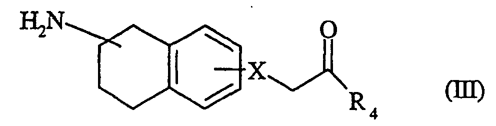 où X et R₄ sont tels que définis ci-dessus et éventuellement en transformant le composé de formule (I) ainsi obtenu en un de ses sels.

Plus particulièrement, la réaction entre les composés de formule (II) et (III) est réalisée dans un solvant organique, tel qu'un alcool inférieur comme le méthanol, l'éthanol et l'isopropanol; le diméthylsulfoxyde (DMSO); un éther linéaire ou cyclique; un amide comme le diméthylformamide (DMF) ou le diméthylacétamide; en utilisant préférablement des quantités au moins équimoléculaires des réactifs.

La température de la réaction est comprise entre la température ambiante et la température de reflux du solvant choisi.

Lorsque R₁ représente l'hydrogène, il est préférable de protéger le groupe fonctionnel par un groupe protecteur afin d'éviter des condensations non souhaitées et de faciliter la condensation souhaitée.

Comme groupes protecteurs, on peut utiliser les groupes protecteurs usuels pour le groupe hydroxy tels que par exemple le méthoxyéthoxyméthyle (MEM) ou le benzyle, selon les techniques bien connues.

De la même façon, les autres groupes sensibles éventuellemnt présents (par exemple R₇=COOH) peuvent être protégés selon les méthodes bien connues.

Les composés de formule (II) sont des composés connus en littérature ou bien ils peuvent être préparés par des procédés analogues aux procédés décrits dans la littérature.

Les composés de formule (III) où R₄ est un groupe -NR₅R₆ et X est CH₂, ainsi que leurs sels er leurs solvates, sont nouveaux et constituent un autre objet de la présente invention. Ces composés, ayant la formule (III') ci dessous, peuvent être préparés selon le schéma 1 suivant 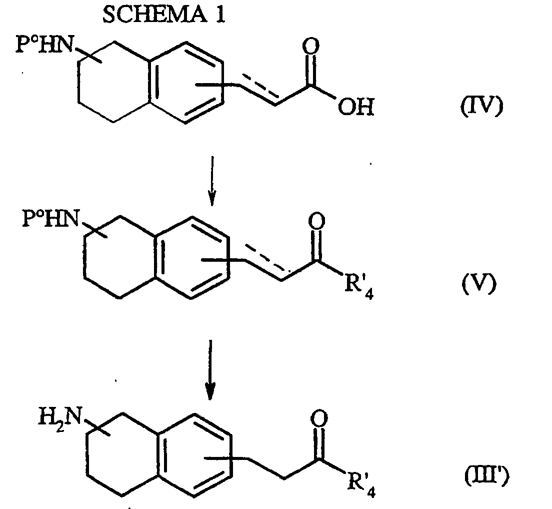 où R'₄ est un groupe -NR₅R₆, NR₅R₆ sont tels que définis ci-dessus, P° est un groupe protecteur et la ligne pointillée représente une liaison double éventuelle.

Comme groupes protecteurs P°, on peut utiliser les groupes protecteurs usuels pour les amines tels que par exemple le *tert*-butoxycarbonyle, l'acétyle, le benzyloxycarbonyle.

Le clivage de ces groupes protecteurs est effectué selon les méthodes habituelles décrites pour le groupe protecteur choisi; dans le cas du *tert*-butoxycarbonyle par exemple, le clivage est normalement effectué par hydrolyse acide.

L'activité des composés de la présente invention vis-à-vis de l'activité bêta-3 a été mise en évidence à l'aide d'essais in vitro sur le colon humain selon la méthode décrite dans EP-B-436435 et dans T. Croci et al, Br. J. Pharmacol., 1997, 122: 139P.

Ces propriétés surprenantes des composés de formule (I) permettent d'envisager leur utilisation comme médicaments à action bêta-3 agoniste.

De plus, les composés de formule (I) sont peu toxiques; notamment, leur toxicité aigüe est compatible avec leur utilisation comme médicaments pour le traitement de maladies dans lesquelles les composés ayant une affinité pour le récepteur bêta-3, notamment les bêta-3 agonistes, trouvent leur application. De telles maladies sont décrites en littérature. Les composés de formule (I), ainsi que leurs sels pharmaceutiquement acceptables, peuvent donc par exemple être indiqués dans le traitement des maladies gastro-intestinales telles que le syndrôme du colon irritable, comme modulateurs de la motricité intestinale, comme lipolytiques, agents anti-obésité, anti-diabétiques, psychotropes, anti-glaucomateux, cicatrisants, anti-dépresseurs, comme inhibiteur des contractions utérines, comme tocolytiques pour prévenir ou retarder les accouchement précoces, pour le traitement et/ou la prophylaxie de la dysménorrhée. En outre les composés de formule (I) peuvent être utilisés dans le traitement de certaines maladies du système nerveux central, telle que par exemple la dépression, ainsi que de certains troubles du système urinaire tel que l'incontinence urinaire.

L'utilisation des composés de formule (I) ci-dessus, ainsi que celle de leurs sels et solvates pharmaceutiquement acceptables pour la préparation de médicaments ci-dessus, constitue un aspect ultérieur de la présente invention.

Pour une telle utilisation, on administre aux mammifères qui nécessitent un tel traitement une quantité efficace d'un composé de formule (I) ou d'un de ses sels et solvates pharmaceutiquement acceptables.

Les composés de formule (I) ci-dessus et leurs sels et solvates pharmaceutiquement acceptables peuvent être utilisés à des doses journalières de 0,01 à 20 mg par kg de poids corporel du mammifère à traiter, de préférence à des doses journalières de 0,1 à 10 mg/kg. Chez l'être humain, la dose peut varier de préférence de 0,5 mg à 1500 mg par jour, notamment de 2,5 à 500 mg selon l'âge du sujet à traiter, le type de traitement, prophylactique ou curatif, et la gravité de l'affection. Les composés de formule (I) sont généralement administrés en unité de dosage de 0,1 à 500 mg, de préférence de 0,5 à 100 mg de principe actif, une à cinq fois par jour.

Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ci-dessus ou un de ses sels et solvates pharmaceutiquement acceptables.

Dans les compositions pharmaceutiques de la présente invention pour l'administration orale, sublinguale, sous-cutanée, intramusculaire, intraveineuse, topique, transdermique ou rectale, les ingrédients actifs de formule (I) ci-dessus, leurs sels et solvates pharmaceutiquement acceptables peuvent être administrés sous formes unitaires d'administration, en mélange avec des supports pharmaceutiques classiques, aux animaux et aux êtres humains, pour le traitement des affections susdites. Les formes unitaires d'administration appropriées comprennent les formes par voie orale telles que les comprimés, les gélules, les poudres, les granulés et les solutions ou suspensions orales, les formes d'administration sublinguale et buccale, les formes d'administration sous-cutanée, intramusculaire ou intraveineuse, les formes d'administration locale et les formes d'administration rectale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granulés dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration locale, on mélange le principe actif dans un excipient pour la préparation de crèmes ou onguents ou on le dissout dans un véhicule pour l'administration intraoculaire, par exemple sous forme de collyre.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les composés de formule (I) notamment les composés (I) marquées par un isotope, peuvent aussi être utilisés comme outils de laboratoire dans des essais biochimiques.

Les composés de formule (I) se lient au récepteur bêta-3-adrénergique. On peut donc utiliser ces composés dans un essai ordinaire de liaison ("binding"), dans lequel on emploie un tissu organique où ce récepteur est particulièrement abondant, et on mesure la quantité de composé (I) déplacé par un composé test, pour évaluer l'affinité dudit composé vis-à-vis des sites de liaison de ce récepteur particulier.

Un autre objet spécifique de la présente invention est donc un réactif utilisable dans les essais biochimiques, qui comprend au moins un composé de formule (I) convenablement marqué.

Les exemples qui suivent illustrent mieux l'invention.

### PREPARATION 1

### Chlorhydrate de (2S)-2-amino-7-pipéridinocarbonylméthoxy-tétraline monohydraté.

### a) (2S)-2-tert-butoxycarbonylamino-7-pipéridinocarbonylméthoxy-tétraline

On mélange à la température ambiante pendant 6 heures 8,1 g (0,025 mole) de (2S)-2-*tert*-butoxycarbonylamino-7-hydroxycarbonylméthoxy-tétraline, 2,5 ml (0,025 mole) de pipéridine 11 g (0,025 mole) de benzotriazol-1-yloxy-tris(diméthylamino)phosphoniumhexafluorophosphate (BOP) et 5,3 ml de triéthylamine dans 250 ml de diméthylformamide. On évapore le solvant, on reprend le résidu dans de l'eau, on extrait à l'acétate d'éthyle, on lave la phase organique avec une solution saturée en NaHCO₃, on filtre sur célite et on lave avec une solution diluée d'acide acétique et avec de l'eau. On sèche et on évapore le solvant sous pression réduite. On purifie le produit par cristallisation dans un mélange acétate d'éthyle/hexane=1/1. On obtient ainsi le produit du titre.
P.f.: 102-103°C.

### b) chlorhydrate de (2S)-2-amino-7-pipéridinocarbonylméthoxy-tétraline monohydraté

On refroidit à +5°C 2,9 g du produit de l'étape précédente dans 56 ml d'acétate d'éthyle et on y ajoute 28 ml d'une solution 3N d'acide chlorhydrique dans de l'acétate d'éthyle. On agite à la température ambiante pendant 24 heures. On isole le composé du titre par filtration sous forme de solide blanc.

### PREPARATION 2

### Chlorhydrate de (2S)-2-amino-7-(n-butylaminocarbonylméthoxy)-tétraline.

### a) (2S)-2-tert-butoxycarbonylamino-7-(n-butylaminocarbonylméthoxy)-tétraline

On agite à la température ambiante pendant 3 heures 4 g de (0,0013 mole) de (2S)-2-*tert*-butoxycarbonylamino-7-hydroxycarbonylméthoxy-tétraline et 1,66 g (0,0143 mole) de N-hydroxysuccinimide dans 40 ml d'acétonitrile et on y ajoute 2,95 g (0,0143 mole) de DCC (dicyclohexylcarbodiimide) dans 15 ml d'éther éthylique. On y ajoute ensuite 1,3 ml (0,0195 mole) de n-butylamine et on agite à la température ambiante pendant une nuit. On évapore le solvant et on réprend le résidu avec de l'eau et on extrait avec de l'acétate d'éthyle. On lave la phase organique avec une solution aqueuse de bicarbonate de sodium, on sèche et on évapore le solvant sous pression réduite. On réprend le résidu dans un mélange acétate d'éthyle/hexane 1:1 on filtre le précipité formé et on le sèche en étuve. On obtient ainsi le produit du titre sous forme de solide blanc.
P.f.: 127-129°C.

### b) chlorhydrate de (2S)-2-amino-7-(n-butylaminocarbonylméthoxy)-tétraline

On refroidit à +5°C un mélange de 1,95 g (0,0054 mole) du produit de l'étape précédente dans 40 ml d'acétate d'éthyle et 20 ml de chlorure de méthylène et on y ajoute 20 ml d'une solution 3N d'acide chlorhydrique dans de l'acétate d'éthyle. On agite à la température ambiante pendant 24 heures. On filtre le précipité ainsi formé qu'on cristallise dans de l'isopropanol. On obtient ainsi le produit du titre.
P.f.: 154-156°C.

### PREPARATION 3

### Chlorhydrate de (2S)-2-amino-7-aminocarbonylméthoxy-tétraline.

### a) (2S)-2-tert-butoxycarbonylamino-7-aminocarbonylméthoxy-tétraline

On agite à la température ambiante sous courant d'azote 0,16 g de NaH à 60% (0,004 mole) et 3,5 ml de DMF anhydre et on y ajoute 1 g (0,0038 mole) de (2S)-2-*tert*-butoxycarbonylamino-7-hydroxy-tétraline dans 9 ml de DMF anhydre et 0,52 g (0,0038 mole) de 2-bromoacétamide dans 2,5 ml de DMF anhydre. On agite à la température ambiante pendant 6 heures et on verse ensuite le mélange dans de l'eau. On filtre le produit précipité et on obtient ainsi le produit du titre.

### b) chlorhydrate de (2S)-2-amino-7-aminocarbonylméthoxy-tétraline

On refroidit à +5°C un mélange de 4,25 g (0,013 mole) du produit de l'étape précédente dans 100 ml d'acétate d'éthyle et 40 ml de méthanol et on y ajoute 53 ml d'une solution 3N d'acide chlorhydrique dans de l'acétate d'éthyle. On agite à la température ambiante pendant 24 heures. On isole le composé du titre par filtration.
P.f. : >250°C.

### PREPARATION 4

### (2S)-2-amino-7-(N,N-diéthylaminocarbonylméthoxy)-tétraline.

### a) (2S)-2-tert-butoxycarbonylamino-7-(N,N-diéthylaminocarbonylméthoxy)-tétraline

On agite à la température ambiante pendant 6 heures 3 g de (0,009 mole) de (2S)-2-*tert*-butoxycarbonylamino-7-hydroxycarbonylméthoxy-tétraline, 0,93 ml de N,N-diéthylamine (0,009 mole), 4 g (0,009 mole) de BOP et 1,9 ml (0,0135 mole) de triéthylamine dans 90 ml de DMF anhydre. On évapore le solvant et on réprend le résidu avec de l'acétate d'éthyle. On lave avec une solution de bicarbonate de sodium. On obtient une émulsion qu'on filtre sur célite. On lave la solution filtrée avec de l'acide acétique et ensuite avec de l'eau. On sèche la phase organique sur du sulfate de sodium et on évapore le solvant sous pression réduite. On purifie le produit par flash-chromatografie en éluant avec un mélange acétate d'éthyle/hexane=1/1. On obtient ainsi le produit du titre sous forme d'huile très dense.

### b) (2S)-2-amino-7-(N,N-diéthylaminocarbonylméthoxy)-tétraline

On refroidit à +5°C un mélange de 2,6 g (0,007 mole) du produit de l'étape précédente dans un mélange de 52 ml d'acétate d'éthyle et 26 ml de chlorure de méthylène et on y ajoute 26 ml d'une solution 3N d'acide chlorhydrique dans de l'acétate d'éthyle. On agite à la température ambiante pendant 24 heures, on évapore le solvant sous pression réduite et on réprend le résidu dans de l'eau, on porte à pH basique avec du NH₄OH, on extrait à l'acétate d'éthyle, on sèche la phase organique et on évapore le solvant sous pression réduite. On purifie le produit brut par chromatographie sur colonne de gel de silice en éluant par un mélange chlorure de méthylène/méthanol=9/1 et on obtient ainsi le produit du titre sous forme d'huile.

### PREPARATION 5

### Chlorhydrate de (2S)-2-amino-7-benzylaminocarbonylméthoxy-tétraline.

### a) (2S)-2-tert-butoxycarbonylamino-7-benzylaminocarbonylméthoxy-tétraline

On mélange 2,26 g (7 mmole) de (2S)-2-*tert*-butoxycarbonylamino-7-hydroxycarbonylméthoxy-tétraline dans 80 ml de DMF et on y ajoute 0,749 g (7 mmole) de benzylamine et 3,1 g de BOP et 1,47 ml (10,5 mmole) de triéthylamine. On agite le mélange à la température ambiante pendant 6 heures et ensuite on laisse reposer sans agitation pendant une nuit. On évapore le solvant, on réprend le résidu avec de l'acétate d'éthyle et on lave avec une solution de bicarbonate de sodium à 5%. On sèche la phase organique et on évapore le solvant sous pression réduite. On obtient ainsi le composé du titre.
P.f. : 135-137°C.
[α]_{D} = - 52,2° (conc. 1% en méthanol).

### b) chlorhydrate de (2S)-2-amino-7-benzylaminocarbonylméthoxy-tétraline

On mélange 2,5 g du produit de l'étape précédente et 20 ml d'acétate d'éthyle et on y ajoute 20 ml d'une solution d'acétate d'éthyle saturée en acide chlorhydrique. On agite à la température ambiante pendant 3 heures et on filtre ensuite le précipité formé. On obtient ainsi le produit du titre.
P.f.: 174-177°C.
[α]_{D} = - 36,7° (conc. 1% en méthanol).

### PREPARATION 6

### (2S)-2-Amino-7-(2-éthoxycarbonyléthyl)-tétraline.

### a) (2S)-2-benzyloxycarbonylamino-7-trifluorométhanesulfonate-tétraline

On dissout 1 g (3,37 mmole) de (2S)-2-benzyloxycarbonylamino-7-hydroxy tétraline dans de la pyridine anhydre. On réfroidit à 0°C et on y ajoute goutte à goutte 0,600 ml (3,7 mmole) d'anhydride triflique. On agite à la température ambiante pendant 4 heures et ensuite on verse dans 20 ml d'eau. On extrait avec de l'éther éthylique, on sèche la phase organique sur du sulfate de sodium et on évapore le solvant sous pression réduite. On purifie le produit brut par chromatographie sur colonne de gel de silice en éluant avec un mélange cyclohexane/acétate d'éthyle=8/2. On obtient ainsi le produit du titre.

### b) (2S)-2-benzyloxycarbonylamino-7-(3-éthoxy-3-oxo-1-propényl)-tétraline

On mélange 4,5 g (10,5 mmole) du produit de l'étape précédente, 2,25 ml de triéthylamine, et 1,36 g (13,65 mmole) d'acrylate d'éthyle et on y ajoute 194 mg (0,74 mmole) de triphénylfosfine. Ensuite en courant d'azote on y ajoute 83 mg de Pd(OAc)₂. On ferme le récipient et après 5 heures à 90°C on y ajoute encore 41 mg de Pd(OAc)₂ et 97 mg de triphénylphosphine et on agite à 90°C pendant 2 heures. On purifie le brut de réaction par flash-chromatographie en éluant par un mélange hexane/acétate d'éthyle =8/2. On obtient ainsi le produit du titre.
P.f.: 119-121°C.
[α]_{D} = - 59,2° (conc. 1% en méthanol).

### c) (2S)-2-amino-7-(2-éthoxycarbonyléthyl)-tétraline

On dissout 1,3 g (34,3 mmole) du produit de l'étape précédente dans 100 ml d'éthanol et on y ajoute 130 mg de Pd/C à 10%. On hydrogène pendant 4 heures à la température ambiante, on filtre le catalyseur et on évapore le solvant sous pression réduite. On obtient 660 mg du produit du titre.

### PREPARATION 7

### (2S)-2-Amino-7-[2-(pipéridinocarbonyl)-éthyl]-tétraline.

### a) (2S)-2-benzyloxycarbonylamino-7-(3-hydroxy-3-oxo-1-propenyl)-tétraline

On dissout 2 g (5,3 mmole) de (2S)-2-benzyloxycarbonylamino-7-(3-éthoxy-3-oxo-1-propenyl)-tétraline dans 20 ml de méthanol et 30 ml de THF et on y ajoute 5,3 ml de NaOH 1N. On agite la solution à la température ambiante pendant une nuit. On évapore le solvant, on réprend le résidu dans de l'eau, on lave à l'éther éthylique, on acidifie la phase aqueuse avec de l'acide citrique à 5% et on extrait avec de l'acétate d'éthyle. On sèche sur du sulfate de sodium et on évapore le solvant. On obtient ainsi le produit du titre.

### b) (2S)-2-benzyloxycarbonylamino-7-(3-oxo-3-pipéridino-1-propényl)-tétraline

En opérant comme décrit dans la Préparation 4 a) mais en utilisant la pipéridine au lieu de la N,N-diéthylamine et le produit de l'étape précédente au lieu de la (2S)-2-*tert*-butoxycarbonylamino-7-hydroxycarbonylméthoxy-tétraline, on obtient le composé du titre.

### c) (2S)-2-amino-7-[2-(pipéridinocarbonyl)-éthyl]-tétraline

On dissout 890 mg (2,2 mmole) du produit de l'étape précédente dans un mélange méthanol/THF 1/1 et on y ajoute 90 mg de Pd/C à 10%. On hydrogène pendant 4 heures, on filtre le catalyseur et on évapore le solvant sous pression réduite. On obtient 500 mg du produit du titre.

### PREPARATION 8

### 4-Benzyloxy-3-(N-propansulfonyl-N-tert-butoxycarbonyl-amino)-1-((2S)-2,3-époxypropoxy)-benzène.

### a) acétate de 4-benzyloxy-3-propansulfonylamino-benzène

On mélange sous atmosphère d'azote 5,0 g (0,00194 mole) d'acétate de 3-amino-4-benzyloxybenzène dans 150 ml de chlorure de méthylène et on y ajoute 3,3 ml (0,0236 mole) de triéthylamine et 2,74 ml (0,0236 mole) de chlorure de 1-propansulfonyle. On agite à la température ambiante pendant une nuit. On lave à l'eau on sépare les deux phases on sèche la phase organique sur du sulfate de sodium on filtre et on évapore le solvant sous pression réduite. On réprend le résidu dans de l'éther isopropylique et on filtre le précipité. On obtient le composé du titre.
P.f.: 135-137°C

### b) acétate de 4-benzyloxy-3-(N-propansulfonyl-N-tert-butoxycarbonylamino)-benzène

On mélange 5,8 g (0,016 mole) du produit de l'étape précédente dans 100 ml de chlorure de méthylène et on y ajoute 4,2 g (0,0192 mole) de di-*tert*-butyldicarbonate et 0,40 g (0,0032 mole) de 4-(N,N-diméthylamino)pyridine. On agite à la température ambiante pendant 1 nuit, on évapore le solvant et on purifie par chromatographie sur colonne de gel de silice en éluant par un mélange cyclohexane/acétate d'éthyle=85/15. On obtient le composé du titre.
P.f. : 83-85°C.

### c) 4-benzyloxy-3-(N-propansulfonyl-N-tert-butoxycarbonyl-amino)-phénol

On mélange 5,4 g (0,0116 mole) du produit de l'étape précédente dans 150 ml de méthanol et on y ajoute 13,9 ml (0,0139 mole) de NaOH 1N. On agite à la température ambiante pendant 30 minutes on ajoute de l'acide citrique jusqu'à pH 6 et on évapore le solvant. On reprend à l'acétate d'éthyle on lave à l'eau on sépare les deux phases on sèche la phase organique sur du sulfate de sodium on filtre et on évapore le solvant sous pression réduite. On obtient le composé du titre.
P.f. : 155-157°C.

### d) 4-benzyloxy-3-(N-propansulfonyl-N-tert-butoxycarbonyl-amino)-1-((2S)-2,3-époxypropoxy)-benzène

On mélange 4,3 g (0,0102 mole) du produit de l'étape précédente dans 100 ml d'acétone et on y ajoute 4,6 g de carbonate de potassium anhydre râpé et 3,3 g (0,0125 mole) de (2S)(+)-glycidyl nosylate. On chauffe au reflux pendant 20 heures, ensuite on filtre, on évapore le solvant et on purifie par chromatographie sur colonne de gel de silice en éluant par un mélange cyclohexane/acétate d'éthyle=75/25. On obtient le composé du titre sous forme d'huile dense.
[a]_{D} = +4,5° (conc. 1% en éthanol).

### EXEMPLE 1

### 1-(3-Méthylsulfonylamino-4-hydroxyphénoxy)-3-(7-éthoxycarbonylméthoxytétralin-2-yl-amino)-2-propanol.

### a) 1-(3-Méthylsulfonylamino-4-benzyloxyphénoxy)-3-(7-éthoxycarbonylméthoxy-tétralin-2-yl-amino)-2-propanol.

On mélange à 40°C pendant 24 heures 1,36 g (0,0032 mole) de 3-N-méthylsulfonylamino-N-tert-butoxycarbonylamino-4-benzyloxy-1-(2,3-époxypropoxy) benzène, 1,04 g (0,0042 mole) de 2-amino-7-éthoxycarbonylméthoxy-tétraline et 0,26 g de LiClO₄ dans 48 ml de CH₃CN. On évapore le solvant, on reprend le résidu avec 30 ml d'acétate d'éthyle, 10 ml de chlorure de méthylène et 20 ml d'une solution 3N d'acide chlorhydrique dans de l'acétate d'éthyle et on agite le mélange à 40°C pendant 6 heures. On évapore le solvant, on y ajoute une solution de NH₄OH jusqu'à pH basique et on extrait à l'acétate d'éthyle. On sèche, on évapore le solvant et on purifie par chromatographie sur colonne de gel de silice en éluant par un mélange chlorure de méthylène/méthanol=9/1. On obtient 1,54 g du produit du titre.

### b) 1-(3-méthylsulfonylamino-4-hydroxyphénoxy)-3-(7-éthoxycarbonylméthoxytétralin-2-yl-amino)-2-propanol

On hydrogène à 40°C à la pression ambiante 0,93 g (0,0015 mole) du produit de l'étape précédente dans 17 ml d'éthanol et 17 ml de tétrahydrofurane avec 0,115 g de Pd/C à 10%. On filtre le catalyseur et on évapore le solvant. On purifie le produit par flash chromatographie sur colonne de gel de silice en éluant par un mélange chlorure de méthylène/méthanol=85/15. On obtient 0,39 g du produit du titre:

### EXEMPLE 2

### 1-(3-Méthylsulfonylamino-4-hydroxyphénoxy)-3-(7-éthoxycarbonylméthoxytétralin-(2S)-2-yl-amino)-(2S)-2-propanol hydraté.

### a) 1-(3-Méthylsulfonylamino-4-benzyloxyphénoxy)-3-(7-éthoxycarbonylméthoxy-tétralin-(2S)-2-yl-amino)-(2S)-2-propanol.

En opérant comme décrit dans l'Exemple 1a) mais en partant des composés optiquement actifs, à savoir le 3-N-méthylsulfonylamino-N-*tert*-butoxycarbonylamino-4-benzyloxy-1-((2S)-2,3-époxypropoxy)-benzène et la (2S)-2-amino-7-éthoxycarbonylméthoxy-tétraline, on obtient le composé du titre.
[α]_{D}= -30,6 ° (conc. 0,5% en méthanol).

### b) 1-(3-méthylsulfonylamino-4-hydroxyphénoxy)-3-(7-éthoxycarbonylméthoxy-tétralin-(2S)-2-yl-amino)-(2S)-2-propanol hydraté

En opérant comme décrit dans l'Exemple 1b) mais en partant du produit de l'étape précédente on obtient le composé du titre.
[α]_{D}= -40,9 °(conc. 1% en méthanol).
¹H RNM 200 MHz (CDCl₃)
1.25 (3H) t (J=7 Hz); 1.45-1.83 (1H) m; 1.90-2.26 (1H) m; 2.35-3.28 (7H) m; 2.82 (3H)s; 3.57-3.96 (2H) m; 4.00-4.31 (1H) m; 4.21 (2H) q (J=7 Hz); 4.51 (2H) s; 6.25-7.07 (6H) m.

### EXEMPLE 3

### 1-(3-Méthylsulfonylamino-4-hydroxyphénoxy)-3-(7-pipéridinocarbonylméthoxy-tétralin-2-yl-amino)-2-propanol.

### a) 1-(3-(méthylsulfonylamino-4-benzyloxyphénoxy)-3-(7-pipéridinocarbonylméthoxy-tétralin-2-yl-amino)-2-propanol.

On chauffe au reflux pendant 8 heures 2,34 g (0,0052 mole) de 3-(N-méthylsulfonyl-N-*tert*-butoxycarbonylamino)-4-benzyloxy-1-(2,3-époxypropoxy)-benzène, 71 ml d'éthanol et 1,44 g (0,0052 mole) de 2-amino-7-pipéridinocarbonylméthoxy-tétraline de la Préparation 1 (base, racémat). On agite à la température ambiante pendant une nuit et après on chauffe encore au reflux pendant 6 heures. On évapore le solvant on reprend le résidu dans 40 ml d'acétate d'éthyle et on y ajoute 32,1 ml d'une solution 3N d'acide chlorhydrique dans de l'acétate d'éthyle. On chauffe au reflux pendant 5 heures et on évapore sous pression réduite. On reprend avec de l'eau, on ajoute NH₄OH jusqu'à pH basique et on extrait à l'acétate d'éthyle. On sèche, on évapore le solvant et on purifie le produit par flash chromatographie sur colonne de gel de silice en éluant par un mélange chlorure de méthylène/méthanol=9/1. On obtient 1,3 g du produit du titre.

### b) 1-(3-méthylsulfonylamino-4-hydroxyphénoxy)-3-(7-pipéridinocarbonylméthoxy-tétralin-2-yl-amino)-2-propanol.

En opérant comme décrit dans l'Exemple 1b) mais en le produit de l'étape précédente on obtient le composé du titre.

### EXEMPLE 4

### 1-(3-Méthylsulfonylamino-4-hydroxyphénoxy)-3-(7-pipéridinocarbonylméthoxy-tétralin-(2S)-2-yl-amino)-(2S)-2-propanol.

En opérant comme décrit dans l'Exemple 3 mais en utilisant utilisant les composés sous forme optiquement active, à savoir le 3-(N-méthylsulfonyl-N-*tert*-butoxycarbonylamino)-4-benzyloxy-1-((2S)-2,3-époxypropoxy)-benzène et la (2S)-2-amino-7-pipéridinocarbonylméthoxy-tétraline de la Préparation 1 (base), on obtient le composé du titre.
¹H RMN 200 MHz (CDCl₃ + D₃COD)
1.37-1.81 (6H) m; 1.96-2.25 (1H) m; 2.50-3.09 (7H) m; 2.87 (3H) s; 3.37-3.57 (4H) m; 3.76-3.96 (2H9 m; 4.01-4.18 (1H) m; 4.57 (2H) bs; 6.41-6.60 (2H) m; 6.66 (1H) dd (J₁=8 Hz, J₂=3 Hz); 6.74 (1H) d (J=9 Hz); 6.86-7.01 (2H) m.

### EXEMPLE 5

### 1-(3-Méthylsulfonylamino-4-hydroxyphénoxy)-3-(7-aminocarbonylméthoxytétralin-(2S)-2-yl-amino)-(2S)-2-propanol.

En opérant comme décrit dans l'Exemple 3 mais en utilisant le 3-(N-méthylsulfonyl-N-*tert*-butoxycarbonylamino)-4-benzyloxy-1-((2S)-2,3-époxypropoxy)-benzène et la (2S)-2-amino-7-aminocarbonylméthoxy-tétraline de la Préparation 3, on obtient le composé du titre.
¹H RNM 200 MHz (DMSO-d₆ + D₂O)
1.60-1.89 (1H) m; 2.06-2.33 (1H) m; 2.57-3.00 (3H) m; 2.93 (3H) s; 3.00-3.32 (3H) m; 3.33-3.53 (1H) m; 3.89 (2H) bd (J=5 Hz); 4.04-4.25 (1H) m; 4.36 (2H) s; 6.59-6.71 (2H) m; 6.75 (1H) dd (J₁=8 Hz, J₂=3 Hz); 6.79-6.92 (2H) m; 7.02 (1H) d (J=9 Hz).

### EXEMPLE 6

### 1-(3-Méthylsulfonylamino-4-hydroxyphénoxy)-3-[7-(N,N-diéthylaminocarbonylméthoxy)-tétralin-(2S)-2-yl-amino]-(2S)-2-propanol.

En opérant comme décrit dans l'Exemple 3 mais en utilisant le 3-(N-méthylsulfonyl-N-*tert*-butoxycarbonylamino)-4-benzyloxy-1-((2S)-2,3-époxypropoxy)-benzène et la (2S)-2-amino-7-(N,N-diéthylaminoaminocarbonylméthoxy)-tétraline de la Préparation 4, on obtient le composé du titre.
¹H RMN 200 MHz (DMSO-d₆ + D₂O)
0.79-1.28 (6H) m; 1.36-1.69 (1H) m; 1.90-2.15 (1H) m; 2.37-3.10 (7H) m; 2.93 (3H) s; 3.12-3.42 (4H) m; 3.73-4.12 (3H) m; 4.64 (2H) bs; 6.39-6.73 (3H) m; 6.73-6.88 (2H) m; 6.95 (1H) d (J=8 Hz).

### EXEMPLE 7

### 1-(3-Méthylsulfonylamino-4-hydroxyphénoxy)-3-[7-(n-butylaminocarbonylméthoxy)-tétralin-(2S)-2-yl-amino]-(2S)-2-propanol.

En opérant comme décrit dans l'Exemple 3 mais en utilisant le 3-(N-méthylsulfonyl-N-*tert*-butoxycarbonylamino)-4-benzyloxy-1-((2S)-2,3-époxypropoxy)-benzène et la (2S)-2-amino-7-(n-butylaminocarbonylméthoxy)-tétraline de la Préparation 2 (base), on obtient le composé du titre.
¹H RMN 200 MHz (CDCl₃ + D₃COD)
1.22 (3H) t (J=7 Hz); 1.08-1.78 (4H) m; 1.89-2.19 (1H) m; 2.38-3.11 (7H) m; 2.84 (3H) s; 3.20-3.42 (2H) m; 3.50-3.76 (1H) m; 3.85 (2H) bs; 3.96-4.17 (1H) m; 4.36 (2H) bs; 6.36-6.58 (2H) m; 6.59-6.70 (1H) m; 6.70-6.86 (1H) m; 6.87-7.10 (2H) m.

### EXEMPLE 8

### 1-(3-Méthylsulfonylamino-4-hydroxyphénoxy)-3-(7-benzylaminocarbonylméthoxy-tétralin-(2S)-2-yl-amino)-(2S)-2-propanol.

En opérant comme décrit dans l'Exemple 3 mais en utilisant le 3-(N-méthylsulfonyl-N-*tert*-butoxycarbonylamino)-4-benzyloxy-1-((2S)-2,3-époxypropoxy)-benzène et la (2S)-2-amino-7-(benzylaminocarbonylméthoxy)-tétraline de la Préparation 5 (base), on obtient le composé du titre.
¹H RMN 200 MHz (CDCl₃ + D₃COD)
1.34-1.69 (1H) m; 1.81-2.17 (1H) m; 2.33-3.10 (7H) m; 2.77 (3H) s; 3.55-3.92 (2H) m; 3.92-4.20 (1H) m; 4.39 (2H) bs; 4.45 (2H) bs; 6.26-6.52 (2H) m; 6.52-6.66 (1H) m; 6.66-6.81 (1H) m; 6.81-7.02 (2H) m; 7.10-7.43 (5H) m.

### EXEMPLE 9

### 1-(3-Propylsulfonylamino-4-hydroxyphénoxy)-3-(7-éthoxycarbonylméthoxytétralin-(2S)-2-yl-amino)-(2S)-2-propanol.

En opérant comme décrit dans l'Exemple 3 mais en utilisant le 4-benzyloxy-3-(N-propansulfonyl-N-*tert*-butoxycarbonyl-amino)-1-((2S)-2,3-époxypropoxy)-benzène de la Préparation 8 et la (2S)-2-amino-7-éthoxycarbonylméthoxy-tétraline, on obtient le composé du titre.
¹H RMN 200 MHz (CDCl₃)
0.84 (3H) t (J=7 Hz); 1.25 (3H) t (J=7 Hz); 1.49-1.93 (3H) m; 1.94-22.8 (1H) m; 2.38-3.41 (9H) m; 3.47-4.01 (2H) m; 4.03-4.36 (1H) m; 4.21 (2H) q (J=7 Hz); 4.39 (2H) bs; 6.07-7.16 (6H) m.

### EXEMPLE 10

### 1-(3-Méthylsulfonylamino-4-hydroxyphénoxy)-3-[7-(2-(pipéridinocarbonyl)-éthyl)-tétralin-(2S)-2-yl-amino]-(2S)-2-propanol.

En opérant comme décrit dans l'Exemple 3 mais en utilisant le 3-(N-méthylsulfonyl-N-*tert*-butoxycarbonylamino)-4-benzyloxy-1-((2S)-2,3-époxypropoxy)-benzène et la (2S)-2-amino-7-[2-(pipéridinocarbonyl)éthyl]-tétraline de la Préparation 7, on obtient le composé du titre.
¹H RMN 200 MHz (DMSO-d₆ + D₂O)
1.26-1.45 (4H) m; 1.45-1.62 (2H) m; 1.62-1.89 (1H) m; 2.09-2.29 (1H) m; 2.34-2.60 (2H) m; 2.61-3.47 (13H) m; 2.93 (3H) s; 3.89 (2H) d (J=5 Hz); 4.05-4.23 (1H) m; 6.68 (1H) dd (J₁=9 Hz, J₂=3 Hz); 6.75-6.89 (2H) m; 6.89-7.14 (3H) m.

### EXEMPLE 11

### 1-(3-Méthylsulfonylamino-4-hydroxyphénoxy)-3-[7-(2-(éthoxycarbonyl)-éthyl)-tétralin-(2S)-2-yl-amino]-(2S)-2-propanol.

En opérant comme décrit dans l'Exemple 3 mais en utilisant le 3-(N-méthylsulfonyl-N-*tert*-butoxycarbonylamino)-4-benzyloxy-1-((2S)-2,3-époxypropoxy)-benzène et la (2S)-2-amino-7-[2-(éthoxycarbonyl)éthyl]-tétraline de la Préparation 6, on obtient le composé du titre.
¹H RMN 200 MHz (CDCl₃)
1.22 (3H) t (J=7 Hz); 1:48-1.71 (1H) m; 1.90-1.22 (1H) m; 2.42-3.21 (11H) m; 2.88 (3H) s; 3.86 (2H) bd; 3.97-4.26 (1H) m; 4.11 (2H) q (J=7 Hz); 6.47 (1H) dd (J₁=9 Hz, J₂=3 Hz); 6.73 (1H) d (J=9 Hz); 6.81-7.14 (4H) m.

### EXEMPLE 12

### Trilfuoroacétate de 1-(3-méthylsulfonylamino-4-benzyloxyphénoxy)-3-(7-hydroxycarbonylméthoxy-tétralin-(2S)-2-yl-amino)-(2S)-2-propanol.

On agite pendant 1 heure une solution de 0,240 g (0,47 mmol) du produit de l'Exemple 2 dans un mélange de 1,5 ml de méthanol et de 1,5 ml d'une solution aqueuse 1N de soude. On acidifie ensuite le milieu réactionnel par addition de résine DOWEX (50 WX2). On lave la résine est successivement 3 fois par de l'eau et 3 fois par du méthanol. On ajoute une solution d'ammoniaque 2N (15ml) dans le méthanol à la résine, et on agite le milieu hétérogène pendant 15 min. On filtre la résine, on la lave deux fois au méthanol puis on la remit sous agitation pendant 15 min avec 15 ml d'une solution d'ammoniaque 2N. Après filtration et lavage de la résine au méthanol, les filtrats sont rassemblés et on évapore les solvant sous pression réduite. On obtient le produit du titre (0.05g) après purification sur HPLC/MS préparative et évaporation des solvants.

### Mode Opératoire de la HPLC/MS

Appareils: Deux pompes Shimatzu LC8 couplées à un spectromètre de masse API 100 PE sciex. Un contrôleur SCL-10A. Un injecteur-collecteur de fractions Gilson 215.
Phase stationnaire : Xterra MS C18, 50x30mm, 5 microm
Phase mobile : Eluant A : H₂O/MeOH 95/5 + CF₃COOH 0.05%
Eluant B : H₂O/MeOH 5/95 + CF₃COOH 0.05%
Débit : 30mL/min
Gradient d'élution :

| | % | % |
|---|---|---|
| t (en min) | A | B |
| 0 | 90 | 10 |
| 3 | 90 | 10 |
| 15 | 10 | 90 |
| 17 | 10 | 90 |
| TR = 7.48 min, [M+H⁺]=481.4. | | |

Le produit purifié a été analysé par HPLC aux conditions suivantes.
Appareils : Deux pompes Shimatzu LC8 couplé à un detecteur UV SPD10-A et à un spectromètre de masse API 100 PE sciex . Un contrôleur SCL-10A. Un injecteur-collecteur de fractions Gilson 215.
Phase stationnaire : Xterra MS C18, 50x4.6mm, 5 microm,
Phase mobile : Eluant A : H₂O/MeOH 95/5 + CF₃COOH 0.05%
Eluant B : H₂O/MeOH 5/95 + CF₃COOH 0.05%
Débit : 3mL/min
Gradient d'élution :

| **t (en min)** | %A | %B |
|---|---|---|
| 0 | 90 | 10 |
| 1 | 90 | 10 |
| 9 | 10 | 90 |
| 10 | 10 | 90 |
| TR = 4.27 min, [M+H⁺]=481.4 | | |

## Revendications

1. Composé de formule (I) : 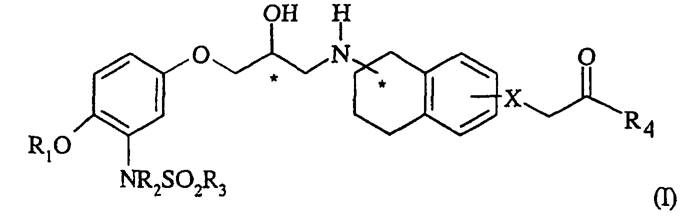 dans laquelle
R₁ et R₂ représentent indépendamment un atome d'hydrogène, un groupe benzyle, benzoyle, un groupe -CO(C₁-C₄)Alk, un groupe CH₂OCH₃, un groupe -COO(C₁-C₄)Alk ou un groupe benzyloxycarbonyle; ou bien
R₁ et R₂ forment ensemble un groupe carbonyle, méthylène ou di(C₁-C₄)Alk-méthylène pour constituer une structure hétérocyclique avec les atomes d'oxygène et d'azote qui les portent ;
R₃ représente un atome d'hydrogène ou un groupe (C₁-C₄)Alk ;
R₄ représente un groupe hydroxy, un groupe (C₁-C₄)alcoxy, ou un groupe -NR₅R₆ ;
R₅ et R₆ représentent indépendamment un atome d'hydrogène; un groupe (C₁-C₄)Alk; un groupe aryle ou hétéroaryle éventuellement substitué par un groupe R₇ ; un groupe aralkyle ou hétéroaralkyle éventuellement substitué par un groupe R₇ ; ou bien R₅ et R₆, avec l'atome d'azote qui les porte, forment un cycle de 5 à 7 atomes, saturé ou insaturé ;
R₇ représente un atome d'hydrogène ou d'halogène, un groupe hydroxy, un groupe (C₁-C₄)Alk, un groupe (C₁-C₄)alcoxy, -COOH, - COO(C₁-C₄)Alk, -CN, -NH(C₁-C₄)Alk ou -N(C₁-C₄)Alk₂ ;
X représente O ou CH₂ ;
et leurs sels ou solvates.

2. Composé selon la revendication 1, où la tétraline est rattachée au groupe amino en position bêta.

3. Composés selon la revendication 1 ou 2, où les deux carbones asymétriques marqués avec " * " ont la configuration (S).

4. Composés selon les revendications 1 à 3, où R₅ et R₆, avec l'atome d'azote qui les porte, représentent un groupe pipéridino, pyrrolidino, morpholino ou thiomorpholino.

5. Composés selon les revendications 1 à 4, où le substituant -X-CH₂-CO-R₄ est rattaché à la position 7 de la tétraline.

6. Composés selon les revendications 1 à 5, où R₅ représente un atome d'hydrogène et R₆ représente un groupe aryle ou hétéroaryle choisi parmi phényle, naphtyle et pyridyle.

7. Composés selon les revendications 1 à 5, où R₅ représente un atome d'hydrogène et R₆ représente un noyau aralkyle ou hétéroaralkyle choisi parmi benzyle, naphtylméthyle et pyridylméthyle.

8. Composé selon la revendication 1, choisi parmi
1-(3-méthylsulfonylamino-4-hydroxyphénoxy)-3-(7-éthoxycarbonylméthoxytétralin-2-yl-amino)-2-propanol;
1-(3-méthylsulfonylamino-4-hydroxyphénoxy)-3-(7-éthoxycarbonylméthoxytétralin-(2S)-2-yl-amino)-(2S)-2-propanol;
1-(3-méthylsulfonylamino-4-hydroxyphénoxy)-3-(7-pipéridinocarbonylméthoxytétralin-2-yl-amino)-2-propanol;
1-(3-méthylsulfonylamino-4-hydroxyphénoxy)-3-(7-pipéridinocarbonylméthoxytétralin-(2S)-2-yl-amino)-(2S)-2-propanol;
1-(3-méthylsulfonylamino-4-hydroxyphénoxy)-3-(7-aminocarbonylméthoxytétralin-(2S)-2-yl-amino)-(2S)-2-propanol;
1-(3-méthylsulfonylamino-4-hydroxyphénoxy)-3-[7-(N,N-diéthylaminocarbonylméthoxy)-tétralin-(2S)-2-yl-amino]-(2S)-2-propanol;
1-(3-méthylsulfonylamino-4-hydroxyphénoxy)-3-[7-(n-butylaminocarbonylméthoxy)-tétralin-(2S)-2-yl-amino]-(2S)-2-propanol;
1-(3-méthylsulfonylamino-4-hydroxyphénoxy)-3-(7-benzylaminocarbonylméthoxytétralin-(2S)-2-yl-amino)-(2S)-2-propanol;
1-(3-propylsulfonylamino-4-hydroxyphénoxy)-3-(7-éthoxycarbonylméthoxytétralin-(2S)-2-yl-amino)-(2S)-2-propanol;
1-(3-méthylsulfonylamino-4-hydroxyphénoxy)-3-[7-(2-(pipéridinocarbonyl)-éthyl)-tétralin-(2S)-2-yl-amino]-(2S)-2-propanol;
1-(3-méthylsulfonylamino-4-hydroxyphénoxy)-3-[7-(2-(éthoxycarbonyl)-éthyl)-tétralin-(2S)-2-yl-amino]-(2S)-2-propanol;
1-(3-méthylsulfonylamino-4-benzyloxyphénoxy)-3-(7-hydroxycarbonylméthoxytétralin-(2S)-2-yl-amino)-(2S)-2-propanol; leurs sels et leurs solvates.

9. Procédé pour la préparation des composés de formule (I) de la revendication 1 **caractérisé en ce qu'**on fait réagir un composé de formule (II) 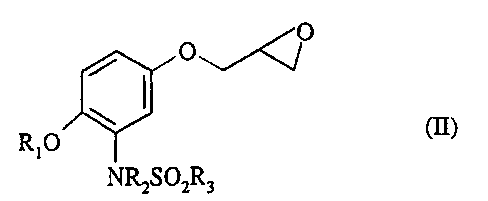 dans laquelle R₁, R₂ et R₃ sont tels qu'indiqués dans la revendication 1, avec une amine de formule (III) 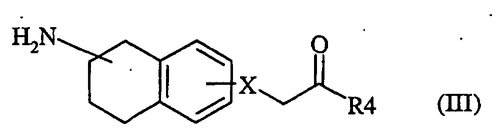 où X et R₄ sont tels que définis dans la revendication 1, et éventuellement transformant le composé de formule (I) ainsi obtenu en un de ses sels.

10. Composition pharmaceutique contenant en tant que principe actif un composé de formule (I) selon les revendications 1 à 8 ou l'un de ses sels pharmaceutiquement acceptables.

11. Médicament contenant en tant que principe actif un composé de formule (I) selon les revendications 1 à 8 ou l'un de ses sels pharmaceutiquement acceptables.

12. Composé de formule (III') 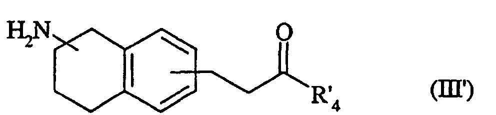 dans laquelle
R'₄ représente un groupe -NR₅R₆ ;
R₅ et R₆ sont tels que définis dans la revendication 1. et leurs sels.

## Patentansprüche

1. Verbindung der Formel (I): 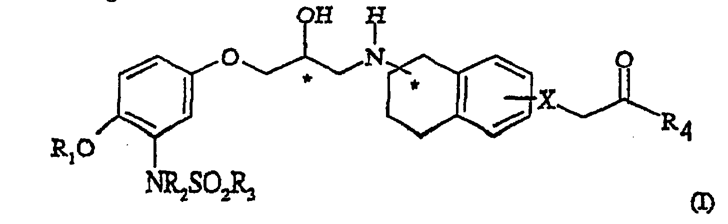 in der
R₁ und R₂ unabhängig voneinander ein Wasserstoffatom, eine Benzylgruppe, eine Benzoylgruppe, eine Gruppe -CO(C₁-C₄)Alk, eine Gruppe -CH₂OCH₃, eine Gruppe -COO(C₁-C₄)Alk oder eine Benzyloxycarbonylgruppe bedeuten; oder
R₁ und R₂ gemeinsam eine Carbonyl-, Methylen- oder Di(C₁-C₄)Alk-methylengruppe bilden und in dieser Weise zusammen mit den sie tragenden Sauerstoff- und Stickstoffatomen eine heterocyclische Struktur bilden;
R₃ ein Wasserstoffatom oder eine Gruppe (C₁-C₄)Alk bedeutet;
R₄ eine Hydroxygruppe, eine (C₁-C₄)Alkoxygruppe oder eine Gruppe -NR₅R₆ darstellt;
R₅ und R₆ unabhängig voneinander ein Wasserstoffatom, eine Gruppe (C₁-C₄)Alk; eine Aryl- oder Heteroarylgruppe, die gegebenenfalls durch eine Gruppe R₇ substituiert ist; eine Aralkyl- oder Heteroaralkylgruppe, die gegebenenfalls durch eine Gruppe R₇ substituiert ist; bedeuten oder R₅ und R₆ zusammen mit dem sie tragenden Stickstoffatom einen gesättigten oder ungesättigten Ring mit 5 bis 7 Atomen bilden;
R₇ ein Wasserstoffatom, ein Halogenatom, eine Hydroxygruppe, eine Gruppe (C₁-C₄)Alk, eine (C₁-C₄)Alkoxygruppe, eine Gruppe -COOH, -COO(C₁-C₄)Alk, -CN, -NH(C₁-C₄)Alk oder -N(C₁-C₄)Alk₂ bedeutet; und
X O oder CH₂ bedeutet;
und deren Salze oder Solvate.

2. Verbindung nach Anspruch 1, worin Tetralin an die Aminogruppe in der Beta-Stellung gebunden ist.

3. Verbindungen nach Anspruch 1 oder 2, worin die beiden mit "*" markierten asymmetrischen Kohlenstoffatome die Konfiguration (S) besitzen.

4. Verbindungen nach den Ansprüchen 1 bis 3, worin R₅ und R₆ zusammen mit dem sie tragenden Stickstoffatom eine Piperidino-, Pyrrolidino-, Morpholino- oder Thiomorpholinogruppe bedeuten.

5. Verbindungen nach den Ansprüchen 1 bis 4, worin der Substituent -X-CH₂-CO-R₄ in der 7-Stellung des Tetralins gebunden ist.

6. Verbindungen nach den Ansprüchen 1 bis 5, worin R₅ ein Wasserstoffatom und R₆ eine Aryl- oder Heteroarylgruppe ausgewählt aus Phenyl, Naphthyl und Pyridyl bedeuten.

7. Verbindungen nach den Ansprüchen 1 bis 5, worin R₅ ein Wasserstoffatom und R₆ einen Aralkyl- oder Heteroaralkyl-Kern ausgewählt aus Benzyl, Naphthylmethyl und Pyridylmethyl bedeuten.

8. Verbindung nach Anspruch 1, ausgewählt aus
1-(3-Methylsulfonylamino-4-hydroxyphenoxy)-3-(7-ethoxycarbonylmethoxy-tetralin-2-yl-amino)-2-propanol;
1-(3-Methylsulfonylamino-4-hydroxyphenoxy)-3-(7-ethoxycarbonylmethoxy-tetralin-(2S)-2-yl-amino)-(2S)-2-propanol;
1-(3-Methylsulfonylamino-4-hydroxyphenoxy)-3-(7-piperidinocarbonylmethoxy-tetralin-2-yl-amino)-2-propanol;
1-(3-Methylsulfonylamino-4-hydroxyphenoxy)-3-(7-piperidinocarbonylmethoxy-tetralin-(2S)-2-yl-aminö)-(2S)-2-propanol;
1-(3-Methylsulfonylamino-4-hydroxyphenoxy)-3-(7-aminocarbonylmethoxy-tetralin-(2S)-2-yl-amino)-(2S)-2-propanol;
1-(3-Methylsulfonylamino-4-hydroxyphenoxy)-3-[7-(N,N-diethylaminocarbonylmethoxy)-tetralin-(2S)-2-yl-amino]-(2S)-2-propanol;
1-(3-Methylsulfonylamino-4-hydroxyphenoxy)-3-[7-(n-butylaminocarbonylmethoxy)-tetralin-(2S)-2-yl-amino]-(2S)-2-propanol;
1-(3-Methylsulfonylamino-4-hydroxyphenoxy)-3-(7-benzylaminocarbonylmethoxytetralin-(2S)-2-yl-amino)-(2S)-2-propanol;
1-(3-Propylsulfonylamino-4-hydroxyphenoxy)-3-(7-ethoxycarbonylmethoxy-tetralin-(2S)-2-yl-amino)-(2S)-2-propanol;
1-(3-Methylsulfonylamino-4-hydroxyphenoxy)-3-[7-(2-(piperidinocarbonyl)-ethyl)-tetralin-(2S)-2-yl-amino]-(2S)-2-propanol;
1-(3-Methylsulfonylamino-4-hydroxyphenoxy)-3-[7-(2-ethoxycarbonyl)-ethyl)-tetralin-(2S)-2-yl-amino]-(2S)-2-propanol;
1-(3-Methylsulfonylamino-4-benzyloxyphenoxy)-3-(7-hydroxycarbonylmethoxy-tetralin-(2S)-2-yl-amino)-(2S)-2-propanol;
und deren Salze und deren Solvate.

9. Verfahren zur Herstellung der Verbindungen der Formel (I) nach Anspruch 1, **dadurch gekennzeichnet, daß** man eine Verbindung der Formel (II) 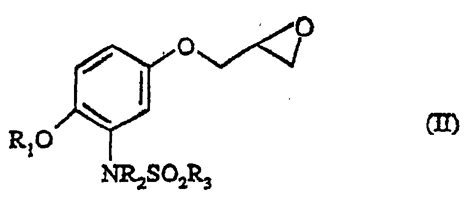 in der R₁, R₂ und R₃ die in Anspruch 1 angegebenen Bedeutungen besitzen, mit einem Amin der Formel (III) 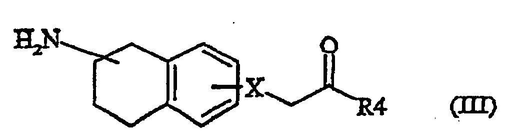 in der X und R₄ die in Anspruch 1 angegebenen Bedeutungen besitzen, umsetzt und gegebenenfalls die in dieser Weise erhaltene Verbindung der Formel (I) in eines ihrer Salze umwandelt.

10. Pharmazeutische Zubereitung enthaltend als Wirkstoff eine Verbindung der Formel (I) nach den Ansprüchen 1 bis 8 oder eines ihrer pharmazeutisch annehmbaren Salze.

11. Arzneimittel enthaltend als Wirkstoff eine Verbindung der Formel (I) nach den Ansprüchen 1 bis 8 oder eines ihrer pharmazeutisch annehmbaren Salze.

12. Verbindung der Formel (III') 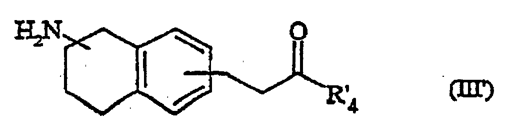 in der
R'₄ eine Gruppe -NR₅R₆ darstellt; und
R₅ und R₆ die in Anspruch 1 angegebenen Bedeutungen besitzen, sowie deren Salze.

## Claims

1. Compound of formula (I): 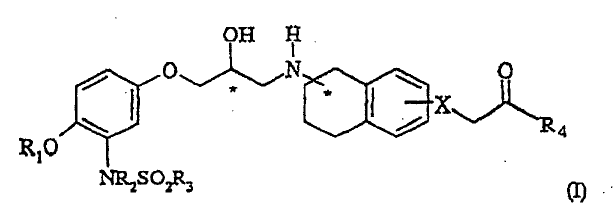 in which:
R₁ and R₂ independently represent a hydrogen atom, a benzyl group, a benzoyl group, a -CO(C₁-C₄)Alk group, a CH₂OCH₃ group, a -COO(C₁-C₄)Alk group or a benzyloxycarbonyl group; or
R₁ and R₂ together form a carbonyl, methylene or di(C₁-C₄)Alk-methylene group to constitute a heterocyclic structure together with the oxygen and nitrogen atoms carrying them;
R₃ represents a hydrogen atom or a (C₁-C₄)Alk group;
R₄ represents a hydroxyl group, a (C₁-C₄)alkoxy group or an -NR₅R₆ group;
R₅ and R₆ independently represent a hydrogen atom; a (C₁-C₄)Alk group; an aryl or heteroaryl group optionally substituted with an R₇ group; an aralkyl or heteroaralkyl group optionally substituted with an R₇ group; or R₅ and R₆, together with the nitrogen atom carrying them, form a saturated or unsaturated 5 to 7 atom cycle;
R₇ represents a hydrogen atom or a halogen atom, a hydroxyl group, a (C₁-C₄)Alk group, a (C₁-C₄)alkoxy group, a -COOH group, a -COO(C₁-C₄)Alk group, a -CN group, an -NH(C₁-C₄)Alk group or an -N(C₁-C₄)Alk₂ group;
X represents O or CH₂;
and its salts or solvates.

2. Compound according to Claim 1, in which the tetralin is attached to the amino group at the beta position.

3. Compound according to Claim 1 or 2, in which the two asymmetric carbon atoms marked with "*" have the (S) configuration.

4. Compound according to Claims 1 to 3, in which R₅ and R₆, together with the nitrogen atom carrying them, represent a piperidino, pyrrolidino, morpholino or thiomorpholino group.

5. Compound according to Claims 1 to 4, in which the substituent -X-CH₂-CO-R₄ is connected to the 7 position of the tetralin.

6. Compound according to Claims 1 to 5, in which R₅ represents a hydrogen atom and R₆ represents an aryl or heteroaryl group selected from phenyl, naphthyl and pyridyl.

7. Compound according to Claims 1 to 5, in which R₅ represents a hydrogen atom and R₆ represents an aralkyl or heteroaralkyl nucleus selected from benzyl, naphthylmethyl and pyridylmethyl.

8. Compound according to Claim 1, selected from:
1-(3-methylsulphonylamino-4-hydroxyphenoxy)-3-(7-ethoxycarbonylmethoxy-tetralin-2-yl-amino)-2-propanol;
1-(3-methylsulphonylamino-4-hydroxyphenoxy)-3-(7-ethoxycarbonylmethoxy-tetralin-(2S)-2-yl-amino)-(2S)-2-propanol;
1-(3-methylsulphonylamino-4-hydroxyphenoxy)-3-(7-piperidinocarbonylmethoxy-tetralin-2-yl-amino)-2-propanol;
1-(3-methylsulphonylamino-4-hydroxyphenoxy)-3-(7-piperidinocarbonylmethoxy-tetralin-(2S)-2-yl-amino)-(2S)-2-propanol;
1-(3-methylsulphonylamino-4-hydroxyphenoxy)-3-(7-aminocarbonylmethoxy-tetralin-(2S)-2-yl-amino)-(2S)-2-propanol;
1-(3-methylsulphonylamino-4-hydroxyphenoxy)-3-(7-(N,N-diethylaminocarbonylmethoxy)-tetralin-(2S)-2-yl-amino]-(2S)-2-propanol;
1-(3-methylsulphonylamino-4-hydroxyphenoxy)-3-[7-(n-butylaminocarbonylmethoxy)-tetralin-(2S)-2-yl-amino]-(2S)-2-propanol;
1-(3-methylsulphonylamino-4-hydroxyphenoxy)-3-(7-benzylaminocarbonylmethoxy-tetralin-(2S)-2-yl-amino)-(2S)-2-propanol;
1-(3-propylsulphonylamino-4-hydroxyphenoxy)-3-(7-ethoxycarbonylmethoxy-tetralin-(2S)-2-yl-amino)-(2S)-2-propanol;
1-(3-methylsulphonylamino-4-hydroxyphenoxy)-3-[7-(2-(piperidinocarbonyl)-ethyl)-tetralin-(2S)-2-yl-amino]-(2S)-2-propanol;
1-(3-methylsulphonylamino-4-hydroxyphenoxy)-3-[7-(2-(ethoxycarbonyl)-ethyl)-tetralin-(2S)-2-yl-amino)-(2S)-2-propanol;
1-(3-methylsulphonylamino-4-benzyloxyphenoxy)-3-(7-hydroxycarbonylmethoxy-tetralin-(2S)-2-yl-amino)-(2S)-2-propanol;
and its salts and its solvates.

9. Process for preparing the compound of formula (I) of Claim 1, **characterized in that** a compound of formula (II): 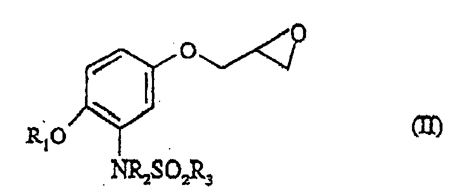 in which R₁, R₂ and R₃ are as defined in Claim 1, is reacted with an amine of formula (III): 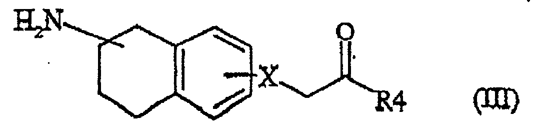 in which X and R₄ are as defined in Claim 1, and the compound of formula (I) thus obtained is optionally transformed into one of its salts.

10. Pharmaceutical composition containing a compound of formula (I) according to Claims 1 to 8 or one of its pharmaceutically acceptable salts as the active principle.

11. Drug containing a compound of formula (I) according to Claims 1 to 8 or one of its pharmaceutically acceptable salts as the active principle.

12. Compound of formula (III'): 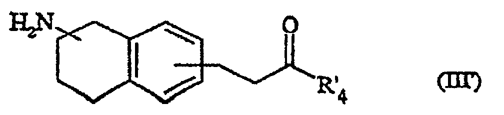 in which:
R'₄ represents an -NR₅R₆ group;
R₅ and R₆ are as defined in Claim 1; and its salts.
